# EUROPÄISCHE PATENTSCHRIFT

(11) **EP 3 873 556 B1**
(45) Veröffentlichungstag und Bekanntmachung des Hinweises auf die Patenterteilung: **21.02.2024**
(21) Anmeldenummer: 19798209.3
(22) Anmeldetag: 29.10.2019
(51) Int. Cl.: A61M 1/16

(54) **VORRICHTUNG FÜR DEN STOFFAUSTAUSCH ZWISCHEN BLUT- UND WENIGSTENS EINEM GAS/GASGEMISCH**
DEVICE FOR EXCHANGING SUBSTANCES BETWEEN BLOOD AND AT LEAST ONE GAS/GAS MIXTURE
DISPOSITIF POUR L'ÉCHANGE DE SUBSTANCES ENTRE DU SANG ET AU MOINS UN MÉLANGE GAZ/GAZ

(30) Priorität: 29.10.2018 DE 102018008459
(43) Veröffentlichungstag der Anmeldung: 08.09.2021
(73) Patentinhaber: enmodes GmbH, 52070 Aachen (DE)
(72) Erfinder: RITTER, Ilse Philine, 52064 Aachen (DE); BORCHARDT, Ralf, 41515 Grevenbroich (DE)
(74) Vertreter: Cohausz Hannig Borkowski Wißgott
(86) Internationale Anmeldenummer: PCT/EP2019/079559
(87) Internationale Veröffentlichungsnummer: WO 2020/089244

(56) Entgegenhaltungen:
- WO-A1-2019/180088
- DE-A1-102016 010 398
- US-A- 5 855 201

## Beschreibung

Die Erfindung betrifft eine Vorrichtung für den Stoffaustausch zwischen Blut- und wenigstens einem Gas/Gasgemisch, umfassend eine erste blutdurchströmbare Kammer, in der eine Vielzahl stoffpermeabler Hohlfasern um ein axial erstrecktes erstes Kernelement herum angeordnet ist, wobei die Hohlfasern von einem Gas/Gasgemisch durchströmbar und von Blut umströmbar sind und weiterhin umfassend eine zweite blutdurchströmbare Kammer, in der eine Vielzahl stoffpermeabler Hohlfasern um ein axial erstrecktes zweites Kernelement herum angeordnet ist, wobei die Hohlfasern von einem Gas/Gasgemisch durchströmbar und von Blut umströmbar sind, wobei die zweite Kammer strömungstechnisch in der Blutströmungsrichtung auf die erste Kammer folgend angeordnet ist, was bedeutet, dass Blut, welches durch die Vorrichtung strömt, zuerst durch die erste Kammer und danach durch die zweite Kammer strömt.

Vorrichtungen für den Stoffaustausch der eingangs genannten Art sind im Stand der Technik grundsätzlich bekannt (siehe z.B. US 5.855.201 (A) oder DE 10 2016 010 398 (A1) und zum Beispiel dafür vorgesehen, um zwischen dem Blut und dem wenigstens einen Gas/Gasgemisch Sauerstoff und CO₂ auszutauschen, nämlich bevorzugt in dem Sinne, dass das Blut in der Kammer mit Sauerstoff angereichert wird und von CO₂ abgereichert wird. Vorrichtungen dieser Wirkungsweise werden häufig auch als Oxygenator bezeichnet.

Die Wirkungsweise solcher Vorrichtungen für den Stoffaustausch beruht darauf, dass in der vom Gas beziehungsweise Gasgemisch durchströmten Hohlfaser und dem Blut unterschiedliche Partialdrücke der auszutauschenden Stoffe vorliegen, so dass durch die stoffpermeablen Hohlfasern hindurch zur Erzielung eines Partialdruckausgleiches die im Ungleichgewicht stehenden Stoffe übertragen werden.

Dies ist möglich aufgrund der stoffpermeablen Ausbildung der Hohlfasern, was es gestattet, dass die für den Austausch vorgesehenen Stoffe, wie in diesem bevorzugten Anwendungsfall Sauerstoff und Kohlendioxid, durch die Hohlfaserwandungen hindurchtreten können. Blut hingegen kann durch die Hohlfaserwandungen nicht aus der Kammer in das Innere einer Hohlfaser übertreten, so dass grundsätzlich über die Hohlfaserwandungen hinweg die blutgefüllte Kammer und die gasgefüllten inneren Bereiche der Hohlfasern bzgl. des Blutes voneinander getrennt sind, insbesondere aber hinsichtlich der Gasbestandteile im Blut über die Hohlfaserwandungen hinweg verbunden sind. Stoffpermeable Hohlfasern, die ein Übertreten von Sauerstoff und Kohlendioxid erlauben, jedoch das Übertreten anderer Blutinhaltsstoffe nicht zulassen, werden auch als semipermeabel bezeichnet.

In Vorrichtungen der bekannten Art und bevorzugt auch bei der erfindungsgemäßen Ausbildung können nebeneinanderliegende Hohlfasern beispielsweise mit Kettfäden zu Matten verbunden sein, so dass die Möglichkeit besteht, solche stoffpermeable Hohlfasern umfassende Matten um ein Kernelement herum aufzuwickeln. Das Kernelement bildet hierbei bevorzugt eine Wickelhilfe und auch im später montierten Zustand einer solchen Vorrichtung ein das gebildete Wickel der Hohlfasern stabilisierendes Element.

Beispielsweise und auch bei der Erfindung bevorzugt können die auf das Kernelement aufgewickelten Hohlfasern in dieser gewickelten Konfiguration gemeinsam in eine jeweilige Kammer eingesetzt werden. Durch ein sogenanntes Vergießen mit einem Klebemittel an den axialen Enden der Kammer ergibt sich ein mit Blut füllbares Volumen der Kammer einerseits und die Möglichkeit, die an den axialen Enden offenen und durch das Vergussklebemittel hindurchreichenden Hohlfasern mit einem Gas bzw. Gasgemisch anzuströmen. Hier sind an den axialen Enden der Kammern die Hohlfasern von dem Vergussklebemittel umgeben. Die Hohlfasern reichen mit Ihren offenen Enden durch das Vergussklebemittel hindurch bis in einen Gaseinlass bzw. Gasauslass.

Bei Vorrichtungen dieser eingangs genannten Art ist es im Wesentlichen bekannt, den Blutfluss und den Gasfluss zumindest im Wesentlichen, bevorzugt also zumindest überwiegend parallel zur axialen Erstreckung der Kammer bzw. des Kernelementes und/oder der Hohlfasern entweder im Gleichstrom oder im Gegenstrom stattfinden zu lassen, wobei beides jeweils für die erzielenden Wirkungen Vorteile und Nachteile mit sich bringt.

So weist ein Gleichstrombetrieb den Vorteil auf, dass sich anfangs ein hoher Konzentrationsgradient und somit auch ein anfangs sehr effizienter Stoffaustausch ergibt, jedoch kann kein vollständiger Austausch erzielt werden.

Im Gegenstrombetrieb ergibt sich der Vorteil, dass eine vollständige Angleichung der Konzentration der auszutauschenden Stoffe bei den beiden Medien möglich ist, jedoch ist der Gegenstrombetrieb verglichen mit dem Gleichstrombetrieb anfangs ineffizient.

Es sind darüber hinaus auch Vorrichtungen dieser genannten Art bekannt, bei denen zwei jeweilige Kammern zum Einsatz kommen, in denen wie eingangs beschrieben, jeweils stoffpermeable Hohlfasern um ein jeweiliges Kammerelement angeordnet sind, wobei die zweite Kammer strömungstechnisch in der Blutströmungsrichtung auf die erste Kammer folgend angeordnet ist. Hierbei kann grundsätzlich erzielt werden, dass in der einen Kammer eine Strömung von Blut und Gas im Gleichstrom und in der anderen Kammer im Gegenstrom erzielt wird.

In diesem bekannten Stand der Technik sind solche zwei Kammern koaxial ineinander angeordnet, was die Konstruktion und insbesondere den Zusammenbau einer solchen Vorrichtung kompliziert macht, insbesondere da unterschiedliche Kern- und Kammerwandungselemente miteinander durch koaxiales Ineinanderstecken kombiniert und hiernach durch Vergießung abgedichtet werden müssen. Eine Vorrichtung dieser Art ist z.B. unter der Bezeichnung "Hilite" von der Firma Xenios bekannt.

Es ist somit eine Aufgabe der Erfindung eine Vorrichtung der eingangs genannten Art mit zwei solchen Kammern bereitzustellen, die konstruktiv einfacher ausgestaltet ist und bevorzugt gegenüber den bekannten Konstruktionen eine ergonomischere Form aufweist. Zudem ist es eine Aufgabe eine gleichmäßigere Durchströmung des Blutes durch den Gasaustauscher zu erreichen und einen effizienteren Stoffaustausch bereitzustellen.

Die Erfindung wird durch die Merkmale des unabhängigen Anspruchs 1 definiert.

Diese Aufgabe wird gemäß der Erfindung dadurch gelöst, dass die erste und die zweite Kammer nebeneinander angeordnet sind. Insbesondere soll dies bedeuten, dass die MittenLängsachsen der beiden Kernelemente sich nicht vollständig überdecken, diese also nicht kollinear liegen, bevorzugt diese Achsen sich auch im Raum nicht schneiden, insbesondere sich zumindest nicht innerhalb der erfindungsgemäßen Vorrichtung schneiden. Beispielsweise kann dies dadurch erreicht werden, dass die Kernelement Mittenachsen, die bevorzugt mit den Kammermittenachsen zusammenfallen, in dieser erfindungsgemäßen Nebeneinander-Anordnung überall entlang der Achserstreckung einen Abstand, zum Beispiel einen radialen Abstand aufweisen, insbesondere wenn die Mittenlängsachsen z.B. mit Abstand parallel verlaufen.

Weiterhin ist es erfindungsgemäß vorgesehen, dass die beiden Kammern in einem axialen Endbereich der jeweiligen Kammererstreckung, eine Verbindung aufweisen, über welche die blutdurchströmbaren Kammervolumina verbunden sind, insbesondere in der Richtung der Beabstandung verbunden sind, insbesondere in radialer Richtung. Bevorzugt liegt die Verbindung bzgl. der Mittenachse in beiden Kammern auf gleicher axialer Höhe bzw. Position, insbesondere axial unmittelbar vor dem Vergussklebemittel.

Eine Ausbildung der Vorrichtung mit dieser Konstruktion hat gegenüber den bekannten Konstruktionen den Vorteil, dass die zwei Kammern nicht koaxial ineinander angeordnet sind und sich hierdurch ein leichterer Zusammenbau und eine insgesamt einfachere Konstruktion ergibt, da für die Herstellung der Vorrichtung es im Wesentlichen lediglich notwendig ist ein auf ein erstes Kammerelement gewickeltes Hohlfaserbündel in die erste Kammer einzusetzen und ein auf ein zweites Kernelement gewickeltes zweites Hohlfaserbündel in eine zweite Kammer einzusetzen, sodass hierdurch eine relative Anordnung der Hohlfaserbündel zueinander entfallen kann.

Durch die Verbindung der Kammervolumina an einem axialen Endbereich beider Kammern wird dabei sichergestellt, dass das Blut aus der ersten Kammer in die zweite Kammer übertreten kann, insbesondere hierbei nach dem Übertritt das Blut seine Strömungsrichtung im Wesentlichen umkehrt.

So kann durch die Verbindung an einem axialen Endbereich, der zum Beispiel durch einen gemeinsamen Bodenbereich einer aufrecht stehenden Vorrichtung ausgebildet sein kann, erreicht werden, dass in einer ersten Kammer das Blut einer solchen Vorrichtung von oben nach unten strömt, sodann am unteren axialen Endbereich von der ersten in die zweite Kammer, zum Beispiel in radialer Richtung, überströmt und sodann in der zweiten Kammer von unten nach oben strömt.

Es kann so beispielsweise in einer bevorzugten Ausführungsform, in der die beiden Kammern und die darin angeordneten auf Kernelemente gewickelten Hohlfasern identisch hinsichtlich ihrer Abmessungen ausgebildet sind, eine exakt gespiegelte Blutführung zwischen den beiden Kammern realisiert werden, so dass sich hierdurch im Vergleich zum Stand der Technik eine deutlich homogenere Behandlung, insbesondere also Sauerstoffanreicherung und Kohlendioxidabreicherung des Blutes erzielt wird.

Bevorzugt sieht die Erfindung vor, dass ein Bluteinlass in die erste Kammer und ein Blutauslass aus der zweiten Kammer an derselben Seite der Vorrichtung angeordnet sind, insbesondere an der Seite der Vorrichtung, welche dem axialen Endbereich mit der Verbindung zwischen den Kammern gegenüberliegt. So wird wie zuvor beschrieben eine Strömungsumkehr nach dem Übertritt zwischen den Kammern beim Blut erzielt, insbesondere mit der benannten Spiegelsymmetrie, sofern die Kammern - wie genannt - hinsichtlich der Abmessungen identisch sind. Die Ausführung ist jedoch auch insoweit bevorzugt als dass der Blutanschluss bei einer stehenden Ausführung von oben erfolgen kann.

In bevorzugter Ausführung sieht die Erfindung weiterhin vor, dass wenigstens ein Gaseinlass und wenigstens ein Gasauslass an sich gegenüberliegenden Seiten der Vorrichtung angeordnet sind, insbesondere nämlich in der Art, dass wenigstens ein Gaseinlass an der Seite angeordnet ist, an welcher auch der Bluteinlass und der Blutauslass der Vorrichtung positioniert sind und wenigstens ein Gasauslass an der gegenüberliegenden Seite, an welcher sich der axiale Endbereich mit der Verbindung zwischen den Kammern befindet. In die jeweiligen Volumina, die vom jeweiligen Gaseinlass und Gasauslass umgrenzt werden, münden die jeweiligen offenen Enden der Hohlfasern, so dass demnach Gaseinlass und Gasauslass in der Hohlfasererstreckungsrichtung, insbesondere der axialen Richtung des Kernelementes beabstandet sind.

Hierdurch kann erzielt werden, dass sowohl in der ersten Kammer als auch in der zweiten Kammer die Gasströmungsrichtung bzgl. der Vorrichtung insgesamt identisch ist, z.B. nämlich bei einem auf einem Boden stehenden Oxygenator von oben nach unten erfolgt, wobei jedoch wie zuvor benannt durch die Anordnung von Bluteinlass und Blutauslass an der der Verbindung gegenüberliegenden Seite in der in Blutströmungsrichtung ersten Kammer das Blut und das Gas/Gasgemisch im Gleichstrom geführt sind und in der in Blutströmungsrichtung zweiten folgenden Kammer das Blut und das Gas/Gasgemisch im Gegenstrom geführt sind.

Hierdurch besteht demnach die Möglichkeit auf konstruktiv besonders einfache und vorteilhafte Weise die Anwendung des Gleich- und Gegenstromprinzips gleichzeitig bei der Blutbehandlung anzuwenden, insbesondere so die jeweiligen Vorteile beider Strömungsprinzipien in der Vorrichtung zu erschließen.

Die Erfindung kann hierbei in einer Ausführungsform vorsehen, dass beide Kammern einen gemeinsamen Gaseinlass und einen gemeinsamen Gasauslass haben. Die beiden Kammern, bzw. die darin angeordneten Hohlfasern werden in ihrem Inneren somit bei dieser Ausführung von derselben Gaszusammensetzung beaufschlagt, insbesondere wobei auch in den Hohlfasern dieselben Strömungsgeschwindigkeiten bei beiden Kammern hinsichtlich des Gases erzielt werden.

Eine andere Ausführungsform der Erfindung kann es jedoch auch vorsehen, dass jede der beiden Kammern jeweils ein eigenes Paar von Gaseinlass und Gasauslass zugeordnet ist, insbesondere wobei also bei jedem Paar der Gaseinlass und der Gasauslass auf gegenüberliegenden Seiten der Vorrichtung angeordnet sind.

Durch eine solche kammerbezogene Trennung der Gasführungen bzw. das jeweilige Vorsehen von Gasanschlusspaaren pro Kammer wird ermöglicht, dass in der ersten Kammer eine andere Gaszusammensetzung verwendet werden kann im Vergleich zur zweiten Kammer. Neben der Möglichkeit unterschiedlicher Zusammensetzungen des Gases erschließt es sich hierdurch auch die Strömungsgeschwindigkeiten verschieden einzustellen bei den beiden Kammern.

Eine konstruktive Vereinfachung eines solchen Gasströmungsprinzips kann sich zum Beispiel dadurch ergeben, dass in der Vorrichtung jeder der beiden Kammern jeweils ein eigener Gaseinlass aber ein gemeinsamer Gasauslass zugeordnet ist. So kann nämlich durch die getrennten Gaseinlässe jede Kammer, bzw. deren Hohlfasern individuell mit Gas angeströmt werden, wobei sich das Gas mit eventueller verschiedener Zusammensetzung oder auch verschiedenen Strömungsgeschwindigkeiten auslassseitig vermischt und durch den gemeinsamen Gasauslass aus der Vorrichtung herausgeleitet wird.

Das erzeugen einer Gasströmung kann auf beliebige Art erzeugt werden, z.B. durch Anschluss eines Gaseinlasses an eine Gasflasche und oder durch Anschluss des Auslasses an eine Pumpe.

Eingangs wurde der erfindungswesentliche Vorteil herausgestellt, dass bei einer gleichdimensionierten, insbesondere abmessungsidentischen Ausbildung von Kammer, Hohlfasern und Kernelement eine im Wesentlichen gespiegelte Behandlung des Blutes in der erfindungsgemäßen Vorrichtung erschlossen wird.

Die Erfindung kann jedoch auch vorsehen, die erste Kammer mit den darin auf einem Kernelement angeordneten Hohlfasern und die zweite Kammer mit den dort auf einem Kernelement angeordneten Hohlfasern verschieden auszubilden, beispielsweise hinsichtlich der Abmessung und/oder der Orientierung der Kammern zueinander bei der erfindungsgemäß vorliegenden räumlichen Nebeneinanderanordnung.

Beispielsweise kann es die Erfindung vorsehen, dass die beiden Kammern unterschiedliche Durchmesser aufweisen, insbesondere die Hohlfaserbündel beider Kammern unterschiedliche Durchmesser haben.

Eine Ausbildung der Kammer mit unterschiedlichen Durchmessern hat insbesondere den Vorteil, dass sich die Strömungsprofile in den Kammern unterschiedlich einstellen lassen. Auch können hier ergonomische oder Design-Aspekte eine Rolle spielen.

Alternativ oder in Kombination mit den vorgenannten Merkmalen besteht auch die Möglichkeit, dass die beiden Kammern unterschiedliche axiale Längen aufweisen, insbesondere die Hohlfaserbündel beider Kammern unterschiedliche axiale Längen haben, bevorzugt hierbei die beiden Kammern, an dem die Verbindung aufweisenden Endbereich jedoch ein gemeinsames Ende aufweisen. Bei einer, wie eingangs benannt, bodenstehenden Vorrichtung können somit die beiden Kammern ein gemeinsames unteres Ende aufweisen, aufgrund der unterschiedlichen Längen können jedoch die oberen Enden eine axiale Beabstandung zueinander haben.

Eine Ausbildung der Kammer mit unterschiedlichen Längen hat insbesondere den Vorteil, dass bezogen auf die gesamten Austauschlänge der Vorrichtung der Wechsel vom Gleichstromprinzip zum Gegenstromprinzip an einer gewünschten Stelle erfolgen kann, die durch die jeweiligen Längen bestimmt ist. Auch kann so die Faseroberfläche verringert und die Effizienz erhöht werden. Ebenso ergibt sich die Möglichkeit hierdurch die Strömungsprofile in den Kammern unterschiedlich zu gestalten und anwendungsbezogen anzupassen. Auch hier können ergonomische und Designaspekte ebenso eine Rolle spielen. Es kann beispielsweise vorgesehen sein, oberhalb einer der beiden Kammern, bevorzugt der kürzeren noch weitere Bauteile anzuordnen.

Durch die Ausbildung der Vorrichtung mit geometrisch verschieden großen Kammern, unabhängig davon, ob dies durch verschiedene Längen und/oder verschiedene Durchmesser erzielt wird, wird im Wesentlichen auch erreicht, dass sich die Kammervolumina von erster Kammer und zweiter Kammer innerhalb der Vorrichtung unterscheiden, so dass bei dem in der gesamten Vorrichtung gleichbleibenden Volumenstrom des Blutes sich für das Blut in der ersten und zweiten Kammern unterschiedliche Verweilzeiten und damit unterschiedliche Behandlungszeiten für den Stoffaustausch ergeben können.

Insbesondere bei einer Ausführungsform, in der die Kammern zumindest gleiche axiale Längen haben und bevorzugt mit den Mittenachsen der Kernelemente parallel angeordnet sind erschließt sich der konstruktiv weiterhin gegebene Vorteil, dass an den axialen Enden der Vorrichtung für den Abschluss der beiden jeweiligen Kammern ein gemeinsames Gehäuseelement der Vorrichtung, insbesondere unten ein Bodenelement und oben ein Deckelelement verwendet werden kann.

Die Erfindung kann grundsätzlich vorsehen, dass die Mittenachsen der Kernelemente insbesondere damit auch die Mittenachsen der Kammern mit Abstand parallel zueinander angeordnet sind. In dieser erfindungsgemäßen Ausführung ergibt sich demnach die Nebeneinander-Anordnung der beiden Kammern durch eine exakt radiale Beabstandung der Kammern zueinander. Insbesondere ist der radiale Abstand zwischen den beiden Mittenachsen größer als die Summe der beiden Radien der jeweiligen Kammern.

Hierdurch sind die Kammerinnenwandungen selbst an dem Ort größter Annäherung, d.h. auf den aufeinander zugewiesenen Seiten der Kammern, noch beabstandet. Die Kammervolumina überschneiden sich demnach in dieser Ausführung nicht und sind nur an dem benannten axialen Endbereich verbunden, nämlich hier in der bevorzugt radialen Verbindungsrichtung.

Eine alternative Ausgestaltung der Erfindung kann es auch vorsehen, dass die Mittenachsen beider Kammern nicht parallel angeordnet sind. Hierbei können die Mittenachsen in einer Projektion betrachtet sich in einem Bereich zwischen der Gaseinlassseite und der Gasauslassseite schneiden oder die Mittenachsen können so angeordnet sein, dass sie in einer Projektion betrachtet sich außerhalb der Vorrichtung, insbesondere jenseits des die Verbindungaufweisenden Endes schneiden. Trotz eines Schnittpunktes in der Projektion schneiden sich die Achsen bevorzugt im Raum tatsächlich jedoch nicht.

Bei einer angenommen bodenstehenden Vorrichtung kann der Schnittpunkt der Mittenachsen somit unterhalb des Bodenbereiches der Vorrichtung und somit außerhalb der Vorrichtung liegen.

Die Ausbildung kann weiterhin derart sein, dass in einer ersten Projektion betrachtet sich die beiden Mittenachsen schneiden und in einer dazu senkrechten Projektion die beiden Mittenachsen parallel liegen. Insbesondere in dieser genannten senkrechten zweiten Projektion weisen demnach die beiden Mittenachsen einen Abstand auf, der zu der beanspruchten Benachbarung der Kammern führt. In der benannten ersten Projektion können die beiden Mittenachsen, sofern diese parallel zueinander angeordnet sind fluchtend hinter einander liegen.

Erfindungsgemäß ist bei allen möglichen Ausbildungen vorgesehen, dass an dem die Verbindung aufweisenden Endbereich um die jeweiligen Hohlfasern jeder Kammer ein Ringraum angeordnet ist, wobei sich die beiden Ringräume beider Kammern im Verbindungsbereich überschneiden. Hieraus erschließt sich, dass durch die Anordnung des Ringraums um die Hohlfasern herum ein solcher Ringraum einen größeren Durchmesser/Querschnittaufweist, als der Durchmesser/Querschnittder Kammer in denjenigen Bereichen, in denen der Ringraum nicht vorgesehen ist. In einem solchen Ringraumbereich kann somit das Blut innerhalb der Kammer auch außen um das auf dem jeweiligen Kernelement liegende Hohlfaserbündel herumströmen und so mit einem geringeren Strömungswiderstand zwischen den Kammern übertreten, als es der Fall wäre, wenn das Blut lediglich durch die Bereiche zwischen den Hohlfasern strömen könnte.

Besonders bevorzugt ist es, wenn in jeder der beiden Kammern sich der freie Querschnitt zwischen den Hohlfasern und der Ringraumwandung in Umfangsrichtung zum Verbindungsbereich hin vergrößert, wodurch im Verbindungsbereich selbst eine gleichmäßige Durchströmung des Blutes erzielt wird, so dass möglichst wenig Scherkräfte auf das Blut im Verbindungsbereich ausgeübt werden.

Es kann weiterhin das Blut vom gesamten Umfang des Hohlfaserbündels in der ersten Kammer in deren Ringraum strömen und bevorzugt an jeder Stelle gleiche Geschwindigkeit aufweisen. Anschließend kann das Blut bevorzugt ohne Be- oder Entschleunigung in den Ringraum der zweiten Kammer überführt werden und von dort gleichmäßig über den gesamten Umfang in das zweite Hohlfaserbündel eintreten. Bevorzugt erfährt das Blut somit zwischen dem Austritt aus dem ersten und dem Eintritt in das zweite Hohlfaserbündel keine Geschwindigkeitsänderung. Stagnationsgebiete und Strömungsverluste können so reduziert werden.

Eine Ausführungsform kann vorsehen, dass in jeder der beiden Kammern sich der freie Querschnitt und/oder der Abstand zwischen den Hohlfasern und der Ringraumwandung im Endbereich ausgehend von einem dem Verbindungsbereich, insbesondere um 180 Grad, gegenüberliegenden Bereich geringsten Querschnitts und/oder Abstandes im Uhrzeigersinn und entgegen dem Uhrzeigersinn bis zum Verbindungsbereich vergrößert.

Eine andere Ausführungsform kann vorsehen, dass in beiden Kammern sich der freie Querschnitt und/oder der Abstand zwischen den Hohlfasern und der Ringraumwandung im Endbereich ausgehend vom Verbindungsbereich im Gleichsinn, insbesondere im Uhrzeigersinn oder gegen den Uhrzeigersinn, bis zurück zum Verbindungsbereich vergrößert , oder aber dass in einer der beiden Kammern sich der freie Querschnitt und/oder der Abstand zwischen den Hohlfasern und der Ringraumwandung im Endbereich ausgehend vom Verbindungsbereich im Uhrzeigersinn und in der anderen Kammer gegen den Uhrzeigersinn jeweils bis zurück zum Verbindungsbereich vergrößert,

Insbesondere bei der letztgenannten Ausführung kann der Verbindungsbereich in einer Richtung senkrecht zur Abstandsrichtung beider Kammern seitlich versetzt neben dem Bereich geringsten Abstandes zwischen den Hohlfasern beider Kammern liegen. Es kann hierbei auch vorgesehen sein, dass ein jeweiliger Ringraum im genannten Bereich geringsten Abstandes zwischen den Hohlfasern beider Kammern eine zusätzliche lokal begrenzte Verjüngung aufweist.

Beispielsweise kann es bei vorgenannten Ausführungen vorgesehen sein, dass der jeweilige Ringraum im Wesentlichen einen Kreisquerschnitt aufweist, dessen Mittelpunkt gegenüber der Mittenachse des im Ringraum befindlichen Kernelementes in Richtung zum Verbindungsbereich versetzt ist, insbesondere radial versetzt ist. Durch die Versetzung und damit die azentrische Anordnung von Ringraum und Kammer wird die Querschnittsvergrößerung des Ringraumes in Richtung zum Verbindungsbereich erzielt.

Ausführungsformen der Erfindung werden anhand der nachfolgenden Figuren näher beschrieben.

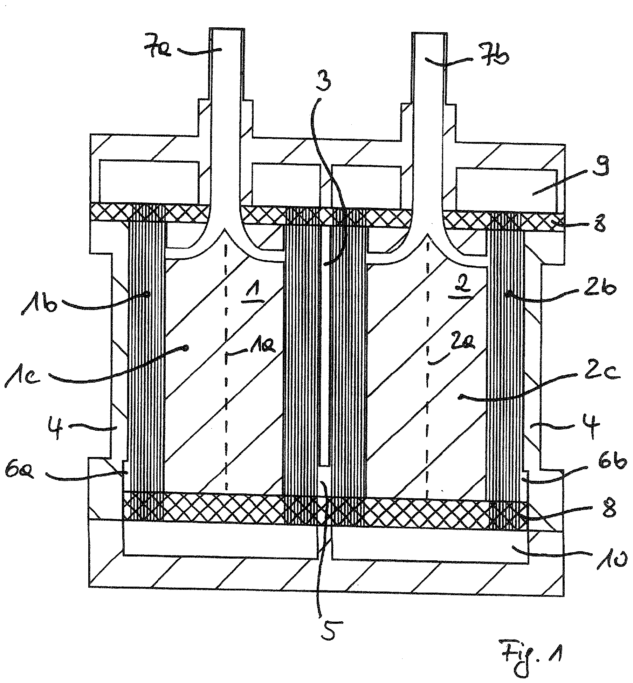

Die Figur 1 zeigt eine bevorzugte Ausführungsform der Erfindung einer Oxygenator-Vorrichtung, die eine erste Kammer 1 und eine zweite Kammer 2 aufweist. Die beiden jeweiligen Kammern weisen axiale Mittenachsen 1a bzw. 2a auf, die in dieser Ausführung parallel zueinander angeordnet sind und aufgrund der Parallelanordnung einen exakt radialen Abstand zueinander aufweisen, wodurch die Nebeneinanderanordnung der ersten und der zweiten Kammer bewirkt wird. Die beiden Kammern 1 und 2 können eine gemeinsame Trennwand 3 im Inneren der Vorrichtung aufweisen, welche die beiden Kammervolumina über den größten Teil der axialen Erstreckung, nämlich bis auf die Verbindung 5 trennt.

Die beiden jeweiligen Kammern 1 und 2 können beispielsweise einen inneren freien Querschnitt, bevorzugt Kreisquerschnitt haben, wobei in der ersten Kammer 1 dieser innere freie Querschnitt gefüllt ist mit einem Hohlfaserbündel 1b, dass auf ein Kernelement 1c aufgewickelt ist und in der zweiten Kammer der innere freie Querschnitt gefüllt ist mit einem Hohlfaserbündel 2b, das auf einem Kernelement 2c aufgewickelt ist. Der innere freie Querschnitt kann über den überwiegenden Teil der axialen Erstreckung der ersten und zweiten Kammer so groß sein wie der äußere Querschnitt des jeweils in der Kammer befindlichen Hohlfaserbündels 1b und 1c, so dass die Hohlfasern faktisch am radial außenliegenden Bereich von den Kammerwandungen 4 kontaktiert sind, die gleichzeitig auch die Gehäuseaußenwandungen der gesamten Vorrichtung bilden. So kann kein Blut radial außen an den Hohlfasern vorbeifließen, abgesehen vom nachfolgend benannten Verbindungsbereich 5.

Am hier axial untenliegenden Endbereich der dargestellten Vorrichtung sind die Kammern 1 und 2 in radialer Richtung durch einen Verbindungsbereich 5 verbunden, der axial stirnseitig vor dem unteren Ende der gemeinsamen Trennwand 3 angeordnet ist.

Über diesen Verbindungsbereich 5 kann in der Kammer 1 hier zum Beispiel von oben nach unten strömendes Blut am axial untenliegenden Ende der Kammer 1 in die Kammer 2 in radialer Richtung überströmen und dann in der zweiten Kammer 2 von unten nach oben strömen.

Der Verbindungsbereich 5 wird hier im Wesentlichen ausgebildet durch den Bereich, in welchem sich zwei jeweilige Ringräume 6a und 6b überschneiden, welche an dem axial untenliegenden Endbereich der Kammern 1,2 das jeweilige Hohlfaserbündel 1b bzw. 2b umgeben.

Die Figur 1 zeigt hierbei eine Ausführung, bei der in den Bluteinlass 7a, der zum Beispiel als Schlauchstutzen ausgebildet ist, Blut in die Kammer 1 einströmen kann. Hierbei wird das Blut über das Innere des Kernelementes 1c radial nach außen in den Bereich zwischen den Hohlfasern des Hohlfaserbündels 1b geleitet und kann hier in axialer Richtung zwischen den Hohlfasern des Bündels 1b nach unten strömen. Nach Übertritt durch den Verbindungsbereich 5 in die zweite Kammer 2 strömt das Blut in axialer Richtung zurück nach oben und kann hier im oberen Endbereich der Kammer 2 über den Blutauslass 7b, der ebenso als Schlauchstutzen mit radial innenliegender Anbindung an das Kammervolumen über das Kernelement die Vorrichtung verlassen.

Die Hohlfaserbündel 1b und 1c sind an den axial gegenüberliegenden Seiten mit einem Vergussklebemittel 8 fest verbunden, wobei das Vergussklebemittel 8 auch die axialen Enden der Kammern 1 und 2 hinsichtlich des Blutvolumens definiert.

Die Hohlfasern reichen in der axialen Richtung durch das Vergussklebemittel 8 hindurch und münden mit ihren offenen Enden bei dieser Vorrichtung im axial obenliegenden Bereich in einem Gaseinlass 9 und im untenliegenden Bereich in einem Gasauslass 10. Hier weisen die beiden Kammern 1 und 2 jeweils einen gemeinsamen Gaseinlass 9 und einen gemeinsamen Gasauslass 10 auf, so dass dasselbe Gas bei dieser Vorrichtung von oben nach unten in der axialen Richtung durch die Hohlfasern geführt wird.

Da der Blutstrom im Hohlfaserbündel der Kammer 1 bei dieser Ausführung axial von oben nach unten geführt ist und in der Kammer 2 von unten nach oben geführt ist ergibt sich eine Führung zwischen Blut und Gas in der Kammer 1, die dem Gleichstromprinzip für den Stoffaustausch entspricht und die in der Kammer 2 dem Gegenstromprinzip für den Stoffaustausch zwischen Blut und Gas entspricht. So kann in dieser hier gezeigten Vorrichtung das Blut durch die Nebeneinanderanordnung der beiden Kammern auf konstruktiv einfache Art und Weise sowohl im Gleich- als auch im Gegenstrom behandelt werden, wobei sich weiterhin durch die hier dargestellte Abmessungsidentität bei Kammer 1 und Kammer 2 eine im Wesentlichen gespiegelte Blutführung zwischen den beiden Kammern 1 und 2 und damit eine besonders homogene Behandlung des Blutes beim Stoffaustausch ergibt.

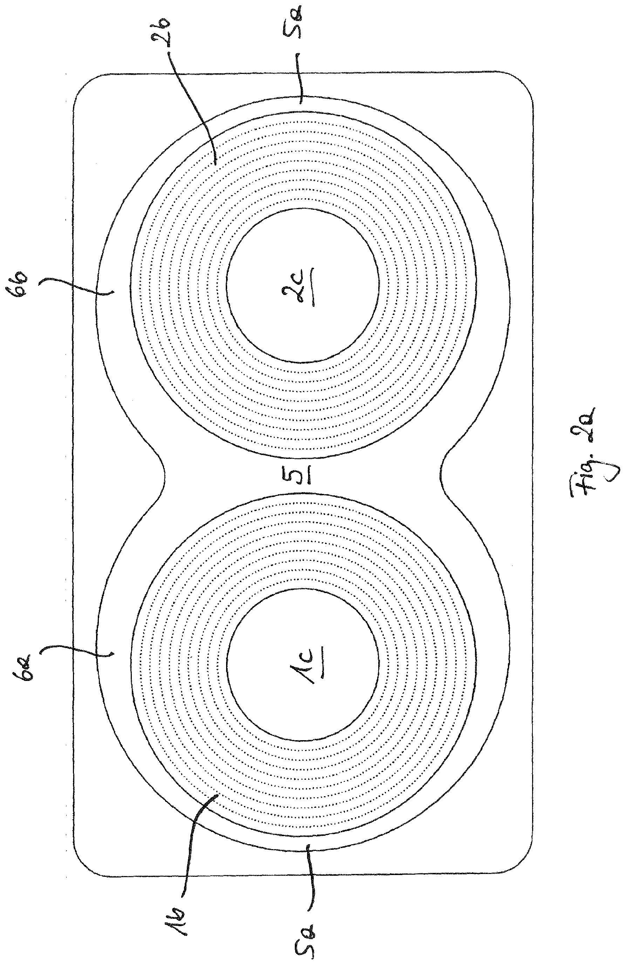

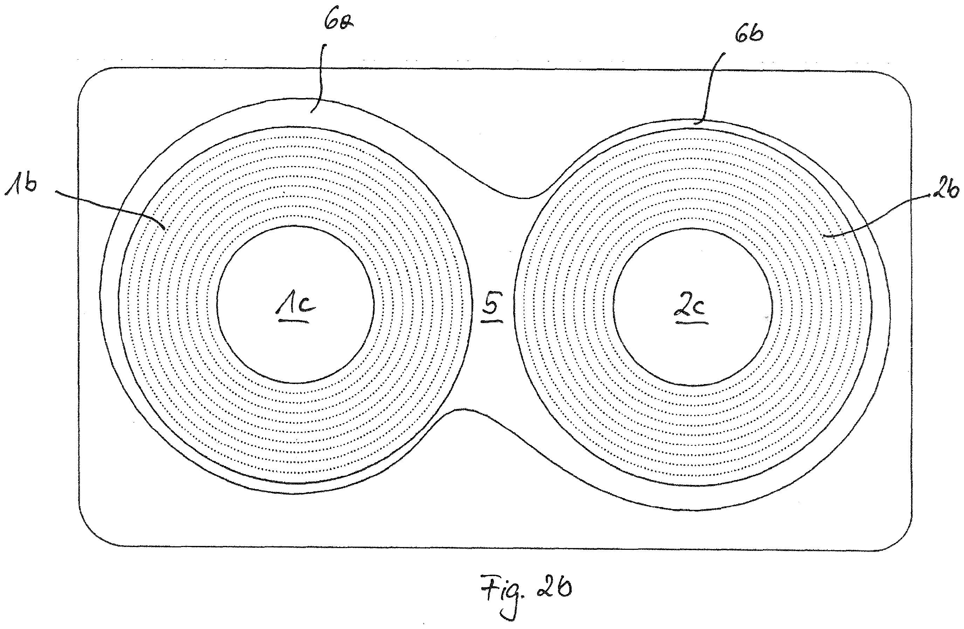

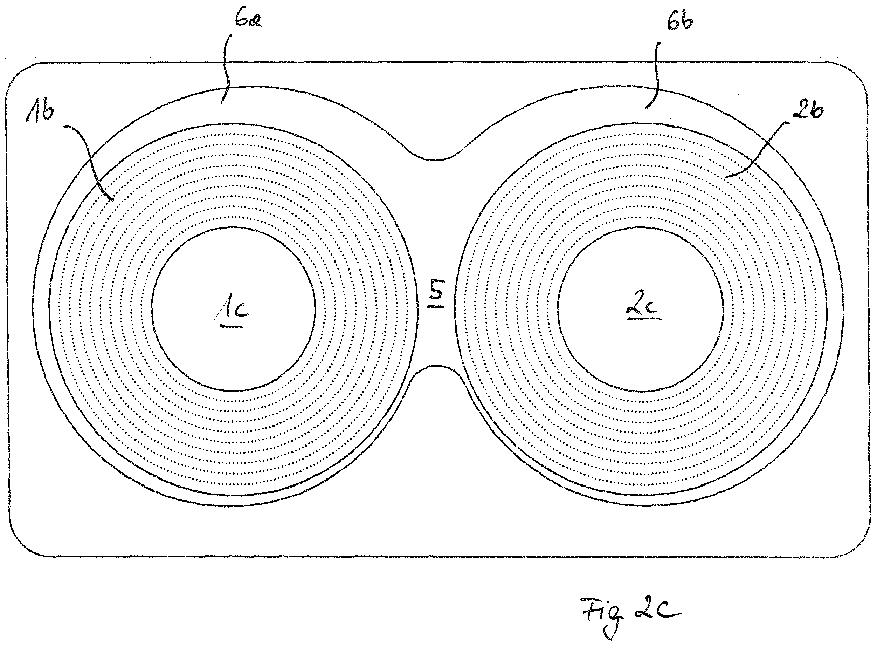

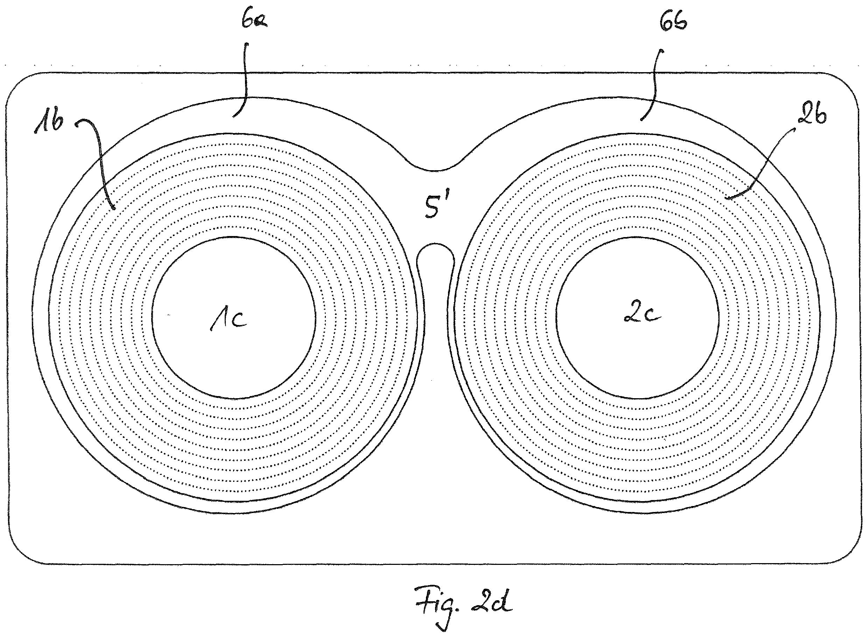

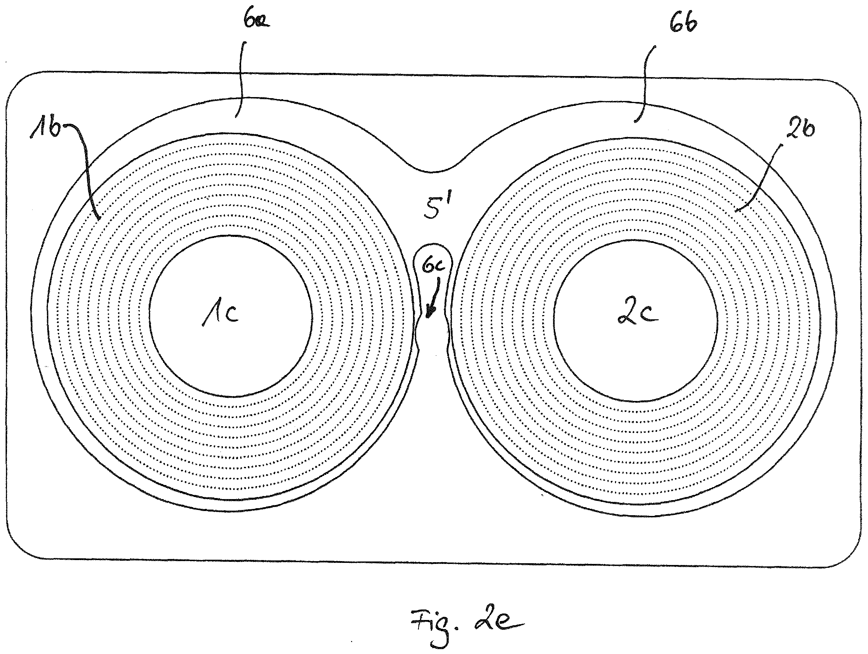

Die Figuren 2 zeigen jeweils eine Schnittdarstellung der erfindungsgemäßen Vorrichtung am bezüglich der Figur 1 axialen unteren Ende durch die Ringräume 6a und 6b, welche die Hohlfaserbündel 1b und 2b von Kammer 1 und Kammer 2 umgeben. Erkennbar ist hier, dass die beiden Ringräume 6a und 6b im Wesentlichen einen kreisförmigen, insbesondere einen Kreisquerschnitt aufweisen können und auf Grund ihrer radialen Beabstandung, die kleiner ist als die Summe der beiden Ringraumradien sich innerhalb der Vorrichtung so überschneiden, dass sich der Verbindungsbereich 5 oder 5' ergibt, in welchem das Blut von der Kammer 1 in die Kammer 2 in im wesentlichen radialer Richtung überströmen kann.

In dem axial bezogen auf die Figur 1 untenliegendem Bereich kann das Blut in im Wesentlichen radialer Richtung aus dem Hohlfaserbündel 1b in den Ringraum 6a übertreten und in diesem mit verringertem Widerstand im Vergleich zur Strömung zwischen den Hohlfasern in der Umfangsrichtung des Ringraumes 6a zum Verbindungsbereich 5 und dort in den Ringraum 6b einströmen und sich wiederum in Umfangsrichtung um das Hohlfaserbündel 2b herum verteilen.

Erkennbar ist hier, dass durch die azentrische Anordnung der hier nicht gezeigten Mittelpunkte der Ringräume und der Kernelementmittenachsen sich innerhalb der Ringräume 6a und 6b in Richtung zum Verbindungsbereich 5 eine Vergrößerung des Querschnittsbereiches ergibt, bevorzugt so, dass der Blutstrom im gesamten Verbindungsbereich eine gleichmäßige Geschwindigkeitsverteilung aufweist.

Bei der Ausführung der Figur 2a vergrößert sich in jeder der beiden Kammern 1, 2 der freie Querschnitt und/oder der Abstand zwischen den Hohlfasern 1b, 2b und der Ringraumwandung im Endbereich ausgehend von einem dem Verbindungsbereich 5 gegenüberliegenden Bereich 5a geringsten Querschnitts und/oder Abstandes im Uhrzeigersinn und entgegen dem Uhrzeigersinn bis zum Verbindungsbereich 5.

Bei der Ausführung der Figur 2b vergrößert sich in beiden Kammern 1,2 der freie Querschnitt und/oder der Abstand zwischen den Hohlfasern 1b, 2b und der Ringraumwandung im Endbereich ausgehend vom Verbindungsbereich 5 im Gleichsinn, hier im Uhrzeigersinn, bis zurück zum Verbindungsbereich 5. Der Verbindungsbereich 5 liegt hier um eine gedachte Linie herum angeordnet, die in Abstandsrichtung beider Kammern 1,2 die beiden Kammermittelpunkte verbindet.

Bei der Ausführung der Figur 2c vergrößert sich in der einen Kammer 1 der freie Querschnitt und/oder der Abstand zwischen den Hohlfasern 1b, 2b und der Ringraumwandung im Endbereich ausgehend vom Verbindungsbereich 5 im Uhrzeigersinn und in der anderen Kammer 2 gegen den Uhrzeigersinn jeweils bis zurück zum Verbindungsbereich 5 und in der Variante der Figur 2d bis zurück zum Verbindungsbereich 5'. Der Verlauf beider Ringräume 6a, 6b kann bevorzugt bei den Ausführungen der Figuren 2a, c, d und e spiegelsymmetrisch zueinander sein. In der Variante der Figur 2d ist der Verbindungsbereich 5' in einer Richtung senkrecht zur Abstandsrichtung beider Kammern 1, 2 seitlich versetzt neben dem Bereich geringsten Abstandes zwischen den Hohlfasern beider Kammern 1,2 angeordnet.

Die Figur 2e zeigt weiterhin eine mögliche Ausführung basierend auf der Ausführung der Figur 2d, gemäß der ein jeweiliger Ringraum 6a, 6b im Bereich geringsten Abstandes zwischen den Hohlfasern beider Kammern 1,2 eine zusätzliche lokal begrenzte Verjüngung 6c aufweist.

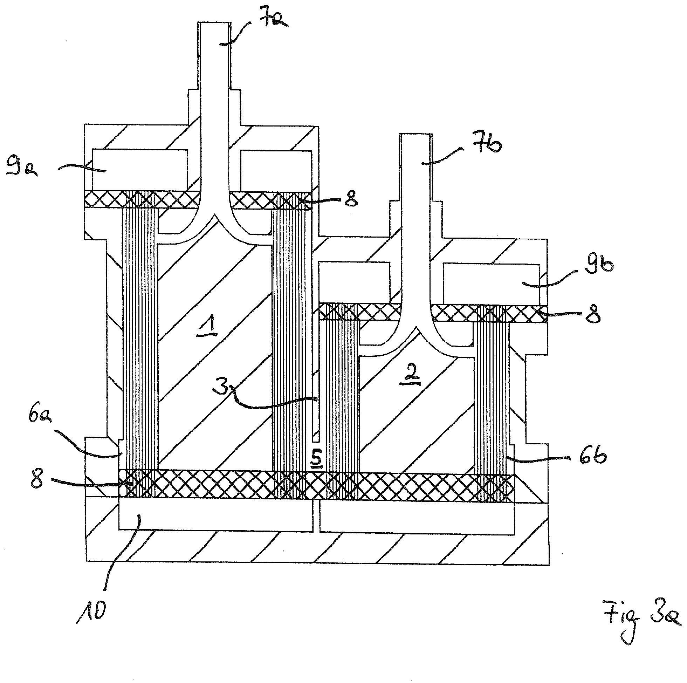

Die Figur 3a zeigt eine weitere Ausführungsmöglichkeit der Erfindung, bei der im Gegensatz zu der Darstellung gemäß Figur 1 die Kammer 1 eine größere axiale Länge aufweist als die Kammer 2, wobei jedoch beide Kammern 1,2 ein hier untenliegendes gemeinsames axiales Ende aufweisen, in welchem der Verbindungsbereich 5, insbesondere mit dem gemäß Figur 2 beschriebenen Ringräumen 6a, 6b realisiert ist. An den axial obenliegenden Endbereichen weist das Gehäuse der Vorrichtung aufgrund der unterschiedlichen axialen Länge von Kammer 1 und Kammer 2 jedoch einen Versatz auf.

Hier kann es beispielsweise vorgesehen sein, dass der Gaseinlass 9a vom Gaseinlass 9b getrennt ist, sodass die beiden Kammern 1,2 auch mit unterschiedlichen Gaszusammensetzungen betrieben werden können. Die Ausführung getrennter Gaseinlässe kann auch bei der Ausführung gemäß Figur 1 vorgesehen sein. Am hier axial untenliegenden Ende ist ein gemeinsamer Gasauslassbereich 10 vorgesehen, in den die Hohlfasern beider Kammern 1,2 durch das Vergussklebemittel 8 hindurch mit Ihren offenen Enden münden.

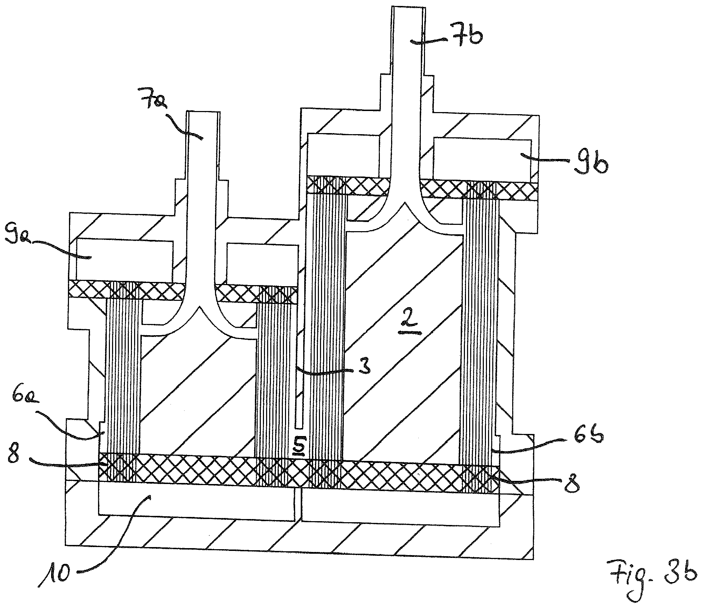

Figur 3b zeigt dieselbe Vorrichtung wie Figur 3a jedoch mit vertauschten Längenverhältnissen der Kammern, d.h. hier ist die axiale Länge der Kammer 1 kleiner als bei der Kammer 2.

Bei den Ausführungen der Figuren 3a und 3b kann es darüber hinaus auch vorgesehen sein, die Gaseinlassbereiche 9a und 9b der beiden Kammern 1 und 2 fluidisch zu verbinden, sodass in beiden Kammern dieselbe Gaszusammensetzung vorliegt.

Ansonsten kann bis auf die axial in der Länge unterschiedliche Ausbildung der beiden Kammern 1,2 die Ausführung der Figuren 3a/b identisch zu der von der Figur 1 sein.

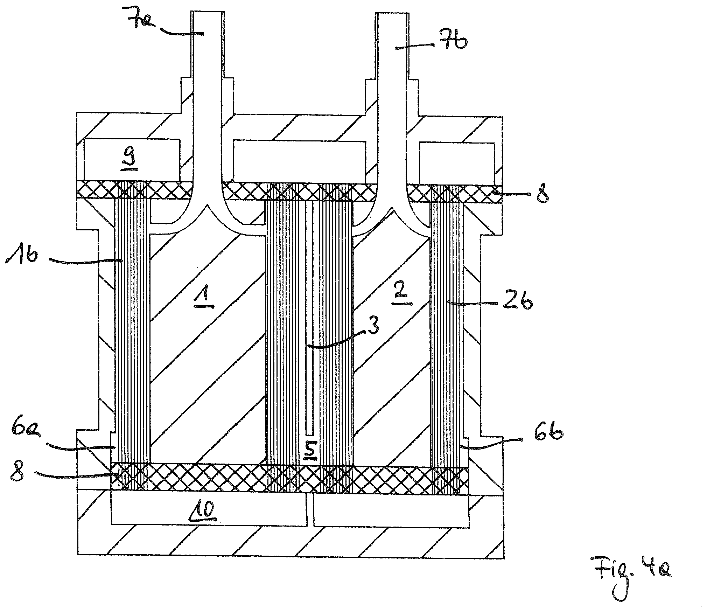

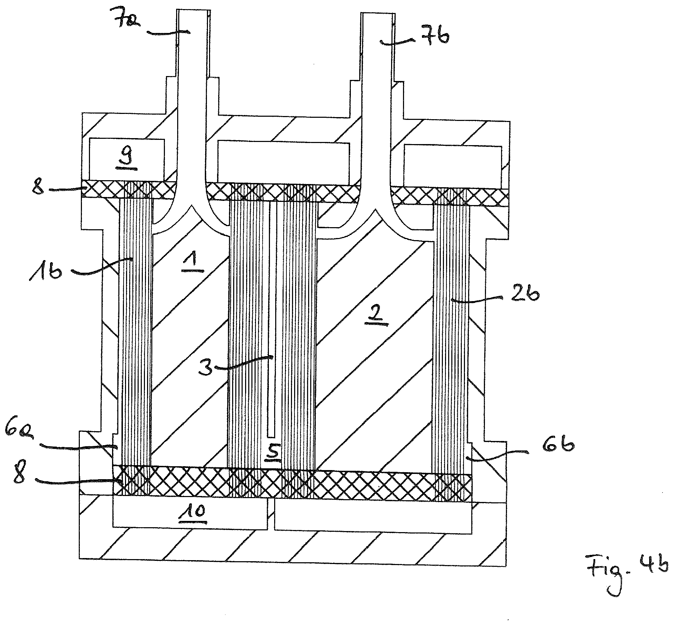

Die Figuren 4a/b visualisieren erfindungsgemäße Ausführung bei denen die Kammern 1 und 2 verschiedene Durchmesser haben. In Figur 4a hat die Kammer 1 gegenüber der Kammer 2 einen geringeren Durchmesser. Bei Figur 4b ist es umgekehrt. Wegen der sich dadurch verschieden ergebenden Volumina der beiden Kammern 1,2 kann in diesen eine unterschiedliche Strömungsgeschwindigkeit des Blutes erzielt werden. Ansonsten sind die Ausführungen der Figuren 4 identisch zu denen der Figuren 1 und 2.

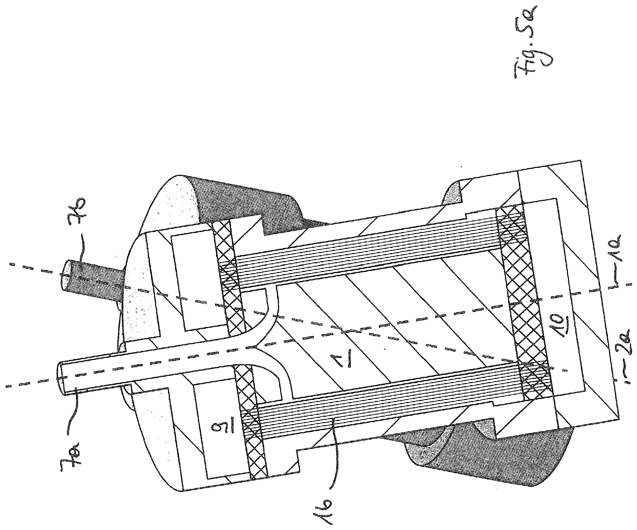

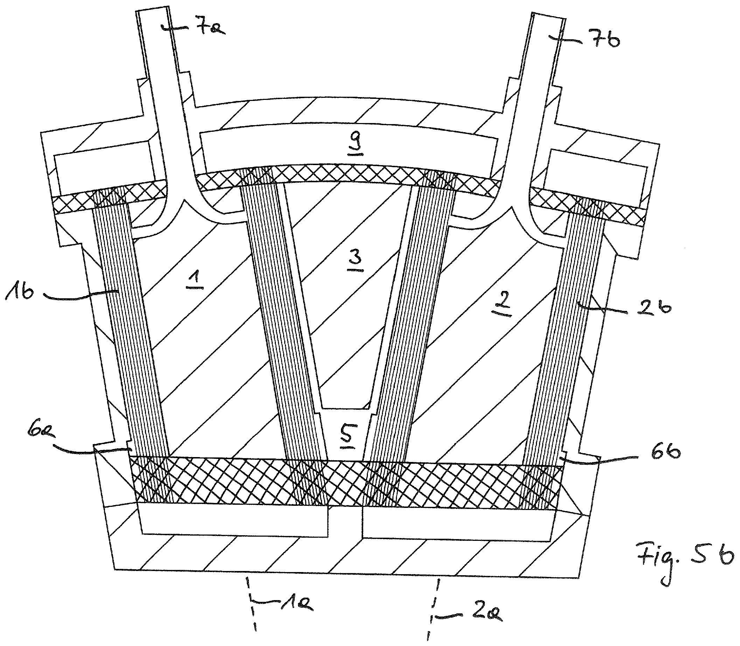

In den Figuren 5a/b ist es visualisiert, dass die beiden Kammern 1 und 2 zueinander nicht parallel verlaufende Mittenachsen 1a, 2a aufweisen können. Dabei kann es gemäß der Figur 5a vorgesehen sein, dass in der hier dargestellten Projektion die beiden Kammermittenachsen 1a und 2a sich innerhalb der Vorrichtung, insbesondere mittig bezogen auf die jeweilige axiale Kammerlänge kreuzen.

Bei der Figur 5b hingegen ist die Ausführung derart, dass die beiden Mittenachsen 1a und 2a sich in dieser Projektionsdarstellung ebenso kreuzen, jedoch der Kreuzungspunkt außerhalb und hier unterhalb der erfindungsgemäßen Vorrichtung liegt. In der Figur 5a weisen somit die Kammern im Wesentlichen in der hier gezeigten Projektion eine X-Konfiguration auf, wohingegen die beiden Kammern in der Figur 5b eine V-Konfiguration haben. Zwischen den beiden Kammern 1 und 2 kann hierbei eine im Querschnitt dieser Ansicht V-förmige Trennwand 3 vorgesehen sein.

In der V-Konfiguration kann die Vorrichtung für beide Kammern einen gemeinsamen planen Bodenbereich aufweisen, wie es hier dargestellt ist, was eine stehende Positionierung der Vorrichtung vereinfacht.

In einer nicht dargestellten Projektion, die senkrecht liegt zur hier visualisierten Aufsichtsrichtung auf die Papierebene, weisen die jeweiligen Mittenachsen bei der Figur 5a 5b eine Beabstandung auf, wodurch die beiden Kammern 1 und 2 auch in dieser Ausführung erfindungsgemäß nebeneinander angeordnet sind. In der gezeigten Ansicht der Figur 5a bedeutet das, dass die Kammern 1 und 2 hintereinander liegen.

## Patentansprüche

1. Vorrichtung für den Stoffaustausch zwischen Blut und wenigstens einem Gas/Gasgemisch, umfassend eine erste blutdurchströmbare Kammer (1), in der eine Vielzahl stoffpermeabler Hohlfasern (1b) um ein axial erstrecktes erstes Kernelement (1c) herum angeordnet ist, wobei die Hohlfasern (1b) von einem Gas / Gasgemisch durchströmbar und von Blut umströmbar sind, und eine zweite blutdurchströmbare Kammer (2), in der eine Vielzahl stoffpermeabler Hohlfasern (2b) um ein axial erstrecktes zweites Kernelement (2c) herum angeordnet ist, wobei die Hohlfasern (2b) von einem Gas / Gasgemisch durchströmbar und von Blut umströmbar sind, wobei die zweite Kammer (2) strömungstechnisch in der Blutströmungsrichtung auf die erste Kammer (1) folgend angeordnet ist, wobei
a. die erste und zweite Kammer (1,2) mit einem Abstand zwischen deren Kernelementmittenachsen (1a, 2a) nebeneinander angeordnet sind und
b. die beiden Kammern (1, 2) in einem axialen Endbereich eine Verbindung (5) aufweisen, über welche die blutdurchströmbaren Kammervolumina in der Richtung der Beabstandung verbunden sind,
**dadurch gekennzeichnet, dass**
c. an dem die Verbindung (5) aufweisenden Endbereich um die jeweiligen Hohlfasern (1b, 2b) jeder Kammer (1, 2) ein Ringraum (6a, 6b) angeordnet ist, wobei sich die beiden Ringräume (6a, 6b) im Verbindungsbereich (5) überschneiden.

2. Vorrichtung nach Anspruch 1, **dadurch gekennzeichnet, dass** ein Bluteinlass (7a) in die erste Kammer (1) und ein Blutauslass (7b) aus der zweiten Kammer (2) an derselben Seite der Vorrichtung angeordnet sind.

3. Vorrichtung nach einem der vorherigen Ansprüche, **dadurch gekennzeichnet, dass** wenigstens ein Gaseinlass (9) und wenigstens ein Gasauslass (10) an sich gegenüberliegenden Seiten angeordnet sind.

4. Vorrichtung nach Anspruch 3, **dadurch gekennzeichnet, dass** beide Kammern (1, 2) einen gemeinsamen Gaseinlass (9) und gemeinsamen Gasauslass (10) haben.

5. Vorrichtung nach Anspruch 3, **dadurch gekennzeichnet, dass** jeder der beiden Kammern (1, 2) jeweils ein eigenes Paar von Gaseinlass (9a, 9b) und Gasauslass (10a, 10b) zugeordnet ist.

6. Vorrichtung nach Anspruch 3, **dadurch gekennzeichnet, dass** jeder der beiden Kammern (1, 2) jeweils ein eigener Gaseinlass (9a, 9b), aber gemeinsamer Gasauslass (10) zugeordnet ist.

7. Vorrichtung nach einem der vorherigen Ansprüche, **dadurch gekennzeichnet, dass** in der in Blutströmungsrichtung ersten Kammer (1) das Blut und das Gas/Gasgemisch im Gleichstrom geführt sind und in der in Blutströmungsrichtung zweiten, folgenden Kammer (2) das Blut und das Gas/Gasgemisch im Gegenstrom geführt sind.

8. Vorrichtung nach einem der vorherigen Ansprüche, **dadurch gekennzeichnet, dass** in jeder der beiden Kammern (1, 2) sich der freie Querschnitt zwischen den Hohlfasern (1b, 2b) und der Ringraumwandung in Umfangsrichtung zum Verbindungsbereich (5) hin vergrößert.

9. Vorrichtung nach einem der vorherigen Ansprüche , **dadurch gekennzeichnet, dass** in jeder der beiden Kammern (1,2) sich der freie Querschnitt und/oder der Abstand zwischen den Hohlfasern (1b, 2b) und der Ringraumwandung im Endbereich ausgehend von einem dem Verbindungsbereich (5) gegenüberliegenden Bereich (5a) geringsten Querschnitts und/oder Abstandes im Uhrzeigersinn und entgegen dem Uhrzeigersinn bis zum Verbindungsbereich (5) vergrößert.

10. Vorrichtung nach einem der vorherigen Ansprüche , **dadurch gekennzeichnet, dass**
a. in beiden Kammern (1,2) sich der freie Querschnitt und/oder der Abstand zwischen den Hohlfasern (1b, 2b) und der Ringraumwandung im Endbereich ausgehend vom Verbindungsbereich im Gleichsinn, insbesondere im Uhrzeigersinn oder gegen den Uhrzeigersinn, bis zurück zum Verbindungsbereich (5) vergrößert, oder
b. in einer der beiden Kammern (1) sich der freie Querschnitt und/oder der Abstand zwischen den Hohlfasern (1b, 2b) und der Ringraumwandung im Endbereich ausgehend vom Verbindungsbereich (5) im Uhrzeigersinn und in der anderen Kammer (2) gegen den Uhrzeigersinn jeweils bis zurück zum Verbindungsbereich (5) vergrößert, insbesondere wobei der Verbindungsbereich (5') in einer Richtung senkrecht zur Abstandsrichtung beider Kammern seitlich versetzt neben dem Bereich geringsten Abstandes zwischen den Hohlfasern beider Kammern liegt.

11. Vorrichtung nach Anspruch 10b, **dadurch gekennzeichnet, dass** ein jeweiliger Ringraum (6a, 6b) im Bereich geringsten Abstandes zwischen den Hohlfasern beider Kammern (1,2) eine zusätzliche lokal begrenzte Verjüngung (6c) aufweist.

12. Vorrichtung nach einem der vorherigen Ansprüche, **dadurch gekennzeichnet, dass** der jeweilige Ringraum (6a, 6b) im Wesentlichen einen Kreisquerschnitt aufweist, dessen Mittelpunkt gegenüber der Mittenachse (1a, 2a) des in dem Ringraum (6a, 6b) befindlichen Kernelementes (1b, 2b) zum Verbindungsbereich (5) versetzt ist, insbesondere radial versetzt ist.

13. Vorrichtung nach einem der vorherigen Ansprüche, **dadurch gekennzeichnet, dass** die beiden Kammern (1, 2) unterschiedliche Durchmesser aufweisen, insbesondere die Hohlfaserbündel (1b, 2b) beider Kammern (1, 2) unterschiedlichen Durchmesser haben.

14. Vorrichtung nach einem der vorherigen Ansprüche, **dadurch gekennzeichnet, dass** die beiden Kammern (1, 2) unterschiedliche axiale Länge aufweisen, insbesondere die Hohlfaserbündel (1b, 2b) beider Kammern (1, 2) unterschiedliche axiale Länge haben, bevorzugt hierbei die beiden Kammern (1, 2) an dem die Verbindung (5) aufweisenden Endbereich ein gemeinsames Ende aufweisen.

15. Vorrichtung nach einem der vorherigen Ansprüche, **dadurch gekennzeichnet, dass** die Mittenachsen (1a, 2a) beider Kammern (1, 2) und/oder Kernelemente (1c, 2c) nicht-parallel angeordnet sind.

16. Vorrichtung nach Anspruch 12, **dadurch gekennzeichnet, dass** die Mittenachsen (1a, 2a) in einer Projektion betrachtet sich in einem Bereich zwischen Gaseinlassseite und Gasauslassseite schneiden oder die Mittenachsen (1a, 2a) in einer Projektion betrachtet sich außerhalb der Vorrichtung, insbesondere jenseits des die Verbindung (5) aufweisenden Endes schneiden.

17. Vorrichtung nach Anspruch 12 oder 13, **dadurch gekennzeichnet, dass** in einer ersten Projektion betrachtet sich die beiden Mittenachsen (1a, 2a) schneiden und in einer dazu senkrechten Projektion die beiden Mittenachsen (1a, 2a) parallel liegen.

## Claims

1. Device for substance exchange between blood and at least one gas/gas mixture, comprising a first chamber (1) which is able to be flowed through by blood and in which a multiplicity of substance-permeable hollow fibres (1b) are arranged around an axially extending first core element (1c), wherein the hollow fibres (1b) are able to be flowed through by a gas/gas mixture and are able to be flowed around by blood, and a second chamber (2) which is able to be flowed through by blood and in which a multiplicity of substance-permeable hollow fibres (2b) are arranged around an axially extending second core element (2c), wherein the hollow fibres (2b) are able to be flowed through by a gas/gas mixture and are able to be flowed around by blood, wherein, in terms of flow, the second chamber (2) is arranged so as to follow the first chamber (1) in the blood-flow direction, wherein
a. the first and second chambers (1, 2) are arranged next to one another with a spacing between their core-element central axes (1a, 2a), and
b. the two chambers (1, 2) have a connection (5) in an axial end region, via which connection the chamber volumes able to be flowed through by blood are connected in the direction of the spacing,
**characterized in that,**
c. at the end region having the connection (5), a ring space (6a, 6b) is arranged around the respective hollow fibres (1b, 2b) of each chamber (1, 2), wherein the two ring spaces (6a, 6b) overlap in the connection region (5).

2. Device according to Claim 1, **characterized in that** a blood inlet (7a) into the first chamber (1) and a blood outlet (7b) out of the second chamber (2) are arranged on the same side of the device.

3. Device according to either of the preceding claims, **characterized in that** at least one gas inlet (9) and at least one gas outlet (10) are arranged on oppositely situated sides.

4. Device according to Claim 3, **characterized in that** the two chambers (1, 2) have a common gas inlet (9) and common gas outlet (10).

5. Device according to Claim 3, **characterized in that** each of the two chambers (1, 2) is assigned in each case one separate pair of gas inlet (9a, 9b) and gas outlet (10a, 10b).

6. Device according to Claim 3, **characterized in that** each of the two chambers (1, 2) is assigned in each case one separate gas inlet (9a, 9b) but a common gas outlet (10).

7. Device according to one of the preceding claims, **characterized in that** the blood and the gas/gas mixture are conducted in a codirectional-flow configuration in the chamber (1) which is first in the blood-flow direction, and the blood and the gas/gas mixture are conducted in a counterflow configuration in the chamber (2) which follows and is second in the blood-flow direction.

8. Device according to one of the preceding claims, **characterized in that** the free cross section between the hollow fibres (1b, 2b) and the ring-space wall increases in a circumferential direction towards the connection region (5) in each of the two chambers (1, 2).

9. Device according to one of the preceding claims, **characterized in that** the free cross section and/or the spacing between the hollow fibres (1b, 2b) and the ring-space wall in the end region, proceeding from a region (5a), situated opposite the connection region (5), of smallest cross section and/or spacing, increases clockwise and anticlockwise as far as the connection region (5) in each of the two chambers (1, 2).

10. Device according to one of the preceding claims, **characterized in that**
a. the free cross section and/or the spacing between the hollow fibres (1b, 2b) and the ring-space wall in the end region, proceeding from the connection region, increases in the same direction, in particular clockwise or anticlockwise, as far as back to the connection region (5) in the two chambers (1, 2), or
b. the free cross section and/or the spacing between the hollow fibres (1b, 2b) and the ring-space wall in the end region, proceeding from the connection region (5), increases clockwise in one of the two chambers (1), and anticlockwise in the other chamber (2), in each case as far as back to the connection region (5), in particular wherein, in a direction perpendicular to the spacing direction between the two chambers, the connection region (5') is situated in a laterally offset manner next to the region of smallest spacing between the hollow fibres of the two chambers.

11. Device according to Claim 10b, **characterized in that** a respective ring space (6a, 6b) has an additional locally delimited narrowing (6c) in the region of smallest spacing between the hollow fibres of the two chambers (1, 2).

12. Device according to one of the preceding claims, **characterized in that** the respective ring space (6a, 6b) has substantially a circular cross section whose centre is offset, in particular is offset radially, in the direction of the connection region (5) in relation to the central axis (1a, 2a) of the core element (1b, 2b) situated in the ring space (6a, 6b) .

13. Device according to one of the preceding claims, **characterized in that** the two chambers (1, 2) have different diameters, in particular the hollow-fibre bundles (1b, 2b) of the two chambers (1, 2) have different diameter.

14. Device according to one of the preceding claims, **characterized in that** the two chambers (1, 2) have different axial length, in particular the hollow-fibre bundles (1b, 2b) of the two chambers (1, 2) have different axial length, the two chambers (1, 2) preferably in this case having a common end at the end region having the connection (5).

15. Device according to one of the preceding claims, **characterized in that** the central axes (1a, 2a) of the two chambers (1, 2) and/or core elements (1c, 2c) are arranged so as to be non-parallel.

16. Device according to Claim 12, **characterized in that** the central axes (1a, 2a) intersect in a region between gas-inlet side and gas-outlet side when seen in a projection, or the central axes (1a, 2a) intersect outside the device, in particular beyond the end having the connection (5), when seen in a projection.

17. Device according to Claim 12 or 13, **characterized in that** the two central axes (1a, 2a) intersect when seen in a first projection and the two central axes (1a, 2a) lie parallel to one another in a projection perpendicular thereto.

## Revendications

1. Dispositif pour l'échange de substances entre du sang et au moins un gaz/mélange de gaz, comprenant une première chambre (1) pouvant être traversée par du sang, dans laquelle une pluralité de fibres creuses (1b) perméables aux substances sont agencées autour d'un premier élément central (1c) s'étendant axialement, les fibres creuses (1b) pouvant être traversées par un gaz/mélange de gaz et pouvant être entourées par du sang, et une deuxième chambre (2) pouvant être traversée par du sang, dans laquelle une pluralité de fibres creuses (2b) perméables aux substances sont agencées autour d'un deuxième élément central (2c) s'étendant axialement, les fibres creuses (2b) pouvant être traversées par un gaz/mélange de gaz et pouvant être entourées par du sang, la deuxième chambre (2) étant agencée, du point de vue de l'écoulement, à la suite de la première chambre (1) dans la direction d'écoulement du sang,
a. la première et la deuxième chambre (1, 2) étant agencées l'une à côté de l'autre avec une distance entre leurs axes médians d'élément central (1a, 2a) et
b. les deux chambres (1, 2) présentant dans une zone d'extrémité axiale une liaison (5) par le biais de laquelle les volumes de chambre pouvant être traversés par du sang sont reliés dans la direction de l'écartement,
**caractérisé en ce que**
c. dans la zone d'extrémité présentant la liaison (5), un espace annulaire (6a, 6b) est agencé autour des fibres creuses respectives (1b·, 2b) de chaque chambre (1, 2), les deux espaces annulaires (6a, 6b) se recoupant dans la zone de liaison (5).

2. Dispositif selon la revendication 1, **caractérisé en ce qu'**une entrée de sang (7a) dans la première chambre (1) et une sortie de sang (7b) de la deuxième chambre (2) sont agencées du même côté du dispositif.

3. Dispositif selon l'une quelconque des revendications précédentes, **caractérisé en ce qu'**au moins une entrée de gaz (9) et au moins une sortie de gaz (10) sont agencées sur des côtés opposés.

4. Dispositif selon la revendication 3, **caractérisé en ce que** les deux chambres (1, 2) ont une entrée de gaz commune (9) et une sortie de gaz commune (10).

5. Dispositif selon la revendication 3, **caractérisé en ce que** chacune des deux chambres (1, 2) est associée à une paire propre d'entrée de gaz (9a, 9b) et de sortie de gaz (10a, 10b).

6. Dispositif selon la revendication 3, **caractérisé en ce que** chacune des deux chambres (1, 2) est associée à une entrée de gaz propre (9a, 9b), mais à une sortie de gaz commune (10).

7. Dispositif selon l'une quelconque des revendications précédentes, **caractérisé en ce que**, dans la première chambre (1) dans la direction d'écoulement du sang, le sang et le gaz/mélange de gaz sont guidés à co-courant et, dans la deuxième chambre (2) suivante dans le direction d'écoulement du sang, le sang et le gaz/mélange de gaz sont guidés à contre-courant.

8. Dispositif selon l'une quelconque des revendications précédentes, **caractérisé en ce que**, dans chacune des deux chambres (1, 2), la section transversale libre entre les fibres creuses (1b, 2b) et la paroi de l'espace annulaire s'agrandit dans la direction circonférentielle vers la zone de liaison (5).

9. Dispositif selon l'une quelconque des revendications précédentes, **caractérisé en ce que**, dans chacune des deux chambres (1, 2), la section transversale libre et/ou la distance entre les fibres creuses (1b, 2b) et la paroi de l'espace annulaire dans la zone d'extrémité s'agrandit dans le sens des aiguilles d'une montre et dans le sens inverse des aiguilles d'une montre jusqu'à la zone de liaison (5) en partant d'une zone (5a) opposée à la zone de liaison (5) de section transversale et/ou de distance la plus faible.

10. Dispositif selon l'une quelconque des revendications précédentes, **caractérisé en ce que**
a. dans les deux chambres (1, 2), la section transversale libre et/ou la distance entre les fibres creuses (1b, 2b) et la paroi de l'espace annulaire dans la zone d'extrémité s'agrandit en partant de la zone de liaison dans le même sens, notamment dans le sens des aiguilles d'une montre ou dans le sens inverse des aiguilles d'une montre, jusqu'à revenir à la zone de liaison (5), ou
b. dans l'une des deux chambres (1), la section transversale libre et/ou la distance entre les fibres creuses (1b, 2b) et la paroi de l'espace annulaire dans la zone d'extrémité s'agrandit en partant de la zone de liaison (5) dans le sens des aiguilles d'une montre et dans l'autre chambre (2) dans le sens inverse des aiguilles d'une montre jusqu'à revenir respectivement à la zone de liaison (5), la zone de liaison (5') étant notamment décalée latéralement dans une direction perpendiculaire à la direction de la distance des deux chambres à côté de la zone de distance la plus faible entre les fibres creuses des deux chambres.

11. Dispositif selon la revendication 10b, **caractérisé en ce qu'**un espace annulaire respectif (6a, 6b) présente un rétrécissement supplémentaire (6c) limité localement dans la zone de la distance la plus faible entre les fibres creuses des deux chambres (1, 2).

12. Dispositif selon l'une quelconque des revendications précédentes, **caractérisé en ce que** l'espace annulaire respectif (6a, 6b) présente essentiellement une section transversale circulaire dont le centre est décalé par rapport à l'axe médian (1a, 2a) de l'élément central (1b, 2b) se trouvant dans l'espace annulaire (6a, 6b) par rapport à la zone de liaison (5), notamment est décalé radialement.

13. Dispositif selon l'une quelconque des revendications précédentes, **caractérisé en ce que** les deux chambres (1, 2) présentent des diamètres différents, notamment les faisceaux de fibres creuses (1b, 2b) des deux chambres (1, 2) ont des diamètres différents.

14. Dispositif selon l'une quelconque des revendications précédentes, **caractérisé en ce que** les deux chambres (1, 2) présentent des longueurs axiales différentes, notamment les faisceaux de fibres creuses (1b, 2b) des deux chambres (1, 2) ont des longueurs axiales différentes, de préférence les deux chambres (1, 2) présentent une extrémité commune au niveau de la zone d'extrémité présentant la liaison (5).

15. Dispositif selon l'une quelconque des revendications précédentes, **caractérisé en ce que** les axes médians (1a, 2a) des deux chambres (1, 2) et/ou des éléments centraux (1c, 2c) sont agencés de manière non parallèle.

16. Dispositif selon la revendication 12, **caractérisé en ce que** les axes médians (1a, 2a), vus dans une projection, se coupent dans une zone entre le côté d'entrée de gaz et le côté de sortie de gaz, ou les axes médians (1a, 2a), vus dans une projection, se coupent à l'extérieur du dispositif, notamment au-delà de l'extrémité présentant la liaison (5).

17. Dispositif selon la revendication 12 ou 13, **caractérisé en ce que,** vus dans une première projection, les deux axes médians (1a, 2a) se coupent et, vus dans une projection perpendiculaire à celle-ci, les deux axes médians (1a, 2a) sont parallèles.
